# FASCICULE DE BREVET EUROPEEN

(11) **EP 0 775 502 B1**
(45) Date de publication et mention de la délivrance du brevet: **18.06.2003**
(21) Numéro de dépôt: 96402497.0
(22) Date de dépôt: 21.11.1996
(51) Int. Cl.: A61N 1/37

(54) **Dispositif médical implantable actif, notamment stimulateur cardiaque de type sentinelle, à discrimination entre signaux parasites et signaux caractéristiques**
Aktive medizinische implantierbare Vorrichtung, insbesondere ein zwischen Stör- und Kennsignalen diskriminierender Bedarfsherzschrittmacher
Active medical implantable device, in particular demand cardiac pacemaker discriminating interference signals and characteristic signals

(30) Priorité: 22.11.1995 FR 9513831
(43) Date de publication de la demande: 28.05.1997
(73) Titulaire: ELA MEDICAL (Société anonyme), F-92541 Montrouge (FR)
(72) Inventeur: Legay, Thierry, 91640 Fontenay Les Briis (FR)
(74) Mandataire: Dupuis-Latour, Dominique

(56) Documents cités:
- EP-A- 0 105 784
- EP-A- 0 321 764
- EP-A- 0 452 732
- EP-A- 0 549 438
- EP-A- 0 580 894
- EP-A- 0 719 568
- DE-A- 3 232 478
- US-A- 4 913 146

## Description

La présente invention concerne les "dispositifs médicaux implantables actifs" tels que définis par la Directive 90/385/CEE du 20 juin 1990 du Conseil des communautés européennes, et plus précisément les stimulateurs cardiaques ou défibrillateurs de type "sentinelle" dont le fonctionnement dépend de la détection de signaux cardiaques spontanément produits par le coeur du patient porteur du dispositif.

Lorsqu'une telle activité cardiaque spontanée est effectivement détectée, et si certaines autres conditions sont remplies, l'appareil peut inhiber la stimulation ou, de façon plus générale, adapter son comportement aux ondes cardiaques spontanées détectées de manière que ce comportement respecte la physiologie du patient ou, à tout le moins, ne crée aucun risque pour lui.

Il est cependant nécessaire, parmi les signaux recueillis, de bien discriminer les "signaux caractéristiques", qui correspondent réellement à des ondes spontanées et qui doivent donc commander une adaptation du comportement du stimulateur, et les "signaux parasites", généralement mêlés aux signaux caractéristiques et captés en même temps que ces derniers.

La discrimination entre signaux caractéristiques et signaux parasites dépend de la cavité cardiaque dans laquelle est opérée la détection.

Dans l'oreillette, les signaux caractéristiques sont ceux d'ondes P, de fibrillation auriculaire ou d'extrasystole, dont les variations d'amplitude peuvent être importantes chez un même patient ; il y a lieu de les distinguer des signaux parasites tels que les ondes de lésion et les ondes de repolarisation d'interface coeur-électrode après stimulation.

Lorsque la détection est opérée dans le ventricule, les signaux caractéristiques sont ceux d'ondes R, de fibrillation ventriculaire et d'extrasystole ventriculaire ; il y a lieu de les distinguer des ondes T, des ondes de lésion et des ondes de repolarisation d'interface coeur-électrode après stimulation.

Si cette discrimination n'est pas opérée de manière convenable, il s'ensuit une "perte de détection" ou "fausse détection" dont les effets ont été largement décrits dans la littérature, avec risque d'induction de malaises chez le porteur du dispositif.

Les stimulateurs (ou défibrillateurs) cardiaques actuels sont tous du type "sentinelle" précité et possèdent donc des moyens pour discriminer les ondes cardiaques spontanées "caractéristiques" des ondes cardiaques "parasites".

Cette discrimination est généralement opérée par filtrage fréquentiel : typiquement, le signal cardiaque endocavitaire subit un filtrage passe-haut avec une fréquence de coupure comprise entre 10 et 30 Hz, permettant ainsi de fortement atténuer les signaux parasites précités, dont le spectre fréquentiel s'étend moins haut que celui des signaux caractéristiques.

Cette technique présente plusieurs inconvénients.

En premier lieu, la fréquence charnière permettant de séparer au mieux les deux domaines spectraux respectifs des ondes caractéristiques et parasites peut varier selon les patients et nécessite donc, si le stimulateur le permet, un réglage particulier après analyse du signal cardiaque du patient.

Une autre difficulté tient au fait que, même si la fréquence charnière est optimisée selon le patient, les signaux parasites laissent subsister un résidu dans la bande de fréquence supérieure, résidu qui peut être gênant lorsque le niveau des signaux caractéristiques est très faible.

De plus, du fait du filtrage passe-haut, les signaux caractéristiques utiles sont partiellement atténués, ce qui rend plus difficile leur détection. En effet, un signal très pur qui ne comprendrait que des ondes caractéristiques, par exemple, dans le cas d'une détection ventriculaire, que des ondes R spontanément émises par le coeur du patient à son rythme propre, par exemple de 60 pulsations par minute, possède la majeure partie de son énergie spectrale en basse fréquence, c'est-à-dire dans le domaine fréquentiel situé au-dessous de 30 Hz, de sorte qu'en filtrant ce domaine on fait perdre au signal une grande partie de son énergie utile.

L'un des buts de la présente invention est de remédier à ces diverses limitations en proposant un traitement de discrimination fondé sur une autre technique que celle du filtrage passe-haut.

L'invention s'applique à un dispositif médical implantable actif du type précité, c'est-à-dire comportant comportant : des moyens détecteurs, pour recueillir le signal cardiaque, spontané ou stimulé, du porteur du dispositif, ce signal cardiaque recueilli comportant une composante de signal caractéristique et des composantes de signaux parasites indésirables ; et des moyens discriminateurs, recevant en entrée le signal cardiaque et délivrant en sortie un signal correspondant essentiellement au signal caractéristique débarrassé des signaux parasites. Un tel dispositif est connu du document DE-A-3 232 478.

De façon caractéristique de l'invention, les moyens discriminateurs comportent des moyens différentiateurs et limiteurs pour évaluer des variations successives du signal cardiaque échantillonné de façon appropriée et délivrer en sortie soit un signal correspondant au signal cardiaque courant si la variation ainsi évaluée est comprise entre des limites positive et négative prédéterminées, soit un signal cardiaque dont la variation est bornée à une valeur prédéterminée, respectivement positive ou négative, dans le cas contraire.

Selon un certain nombre de caractéristiques avantageuses:
- les moyens détecteurs comportent des moyens pour échantillonner et numériser le signal cardiaque détecté et les moyens différentiateurs et limiteurs établissent les valeurs successives de pente du signal cardiaque par évaluation d'incréments entre deux valeurs d'échantillonnage successives ;
- les moyens différentiateurs et limiteurs retranchent du signal numérisé courant non borné la valeur précédente éventuellement bornée de ce signal et comparent la valeur absolue de la différence résultante à une valeur prédéterminée de pente discriminante ; dans ce cas, avantageusement, lorsque la valeur absolue de la différence résultante est supérieure à la valeur de pente discriminante, les moyens différentiateurs et limiteurs ajoutent ou retranchent, selon le cas, à la valeur précédente, éventuellement bornée, du signal numérisé courant un incrément donné égal à ladite valeur prédéterminée positive ou négative et délivrent en sortie une valeur traitée correspondante ;
- la limite positive et/ou la limite négative des moyens limiteurs de pente sont des valeurs ajustables, notamment en fonction de l'évolution du signal cardiaque détecté ;
- la valeur de pente discriminante est une valeur ajustable, notamment en fonction de l'évolution du signal cardiaque détecté.

On va maintenant décrire plus en détail l'invention, en référence aux dessins annexés.

La figure 1 est une représentation sous forme de schéma par blocs des fonctions mises en oeuvre par le dispositif de l'invention.

La figure 2 est un organigramme illustrant le traitement de signal selon l'invention réalisé par voie logicielle.

La figure 3 illustre, à partir d'un même signal cardiaque recueilli, la différence entre un circuit de discrimination classique à filtrage passe-haut et un circuit selon l'invention.

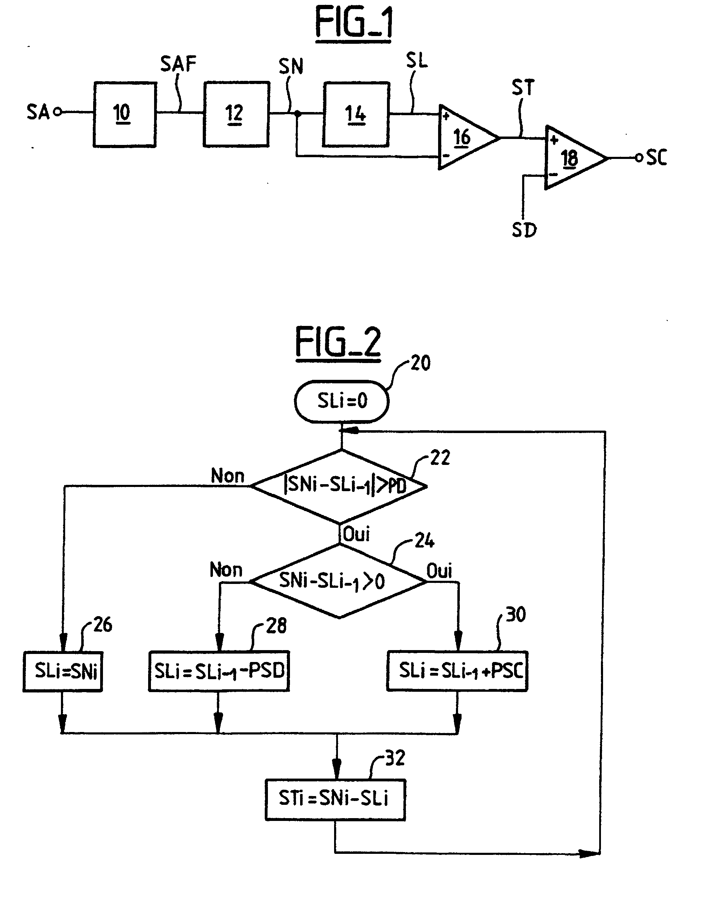

Sur la figure 1, on a représenté schématiquement, sous forme d'une chaîne de blocs fonctionnels, le principe mis en oeuvre par l'invention.

L'idée de base de l'invention consiste à utiliser non pas un filtrage fréquentiel du signal recueilli, mais un suivi de la pente de ce signal, cette pente étant définie comme la dérivée première du signal, dérivée qui peut être approximée de façon satisfaisante dans le cas d'un signal échantillonné par la valeur de l'accroissement du signal durant un laps de temps fixe prédéterminé, typiquement la durée séparant la détermination de deux échantillons successifs.

L'idée de base de l'invention repose sur la constatation de l'existence d'une pente limite ou pente discriminante distinguant les signaux caractéristiques des signaux parasites. On constate en effet que les signaux caractéristiques ont des pentes notablement supérieures à celles des signaux parasites, de sorte que le suivi du signal par analyse de sa pente permet de détecter la présence ou non d'un signal caractéristique, et donc de commander ou non une action particulière du stimulateur, par exemple l'inhibition de la stimulation.

Le signal cardiaque analogique SA recueilli par l'électrode endocavitaire est tout d'abord traité par un étage 10, qui procède notamment à un filtrage préalable tel qu'un filtrage passe-bas éliminant tous les signaux situés au-dessus d'une fréquence donnée, par exemple 500 Hz, au-delà de laquelle on ne retrouve pas de spectre de signaux caractéristiques. Le signal analogique filtré SAF résultant est alors soumis au traitement selon l'invention, qui peut être réalisé soit sous forme analogique, soit sous forme numérique, notamment par logiciel.

Dans la forme la plus simple, le signal analogique filtré SAF est appliqué à un étage numériseur 12 délivrant un signal numérisé SN ; l'échantillonnage du signal est réalisé à une fréquence compatible avec la bande spectrale, donc à une fréquence d'au moins le double de la fréquence de coupure haute du filtre passe-bas de l'étage 10.

Le signal numérisé SN subit alors, par un étage 14, une différentiation (c'est-à-dire, comme indiqué plus haut, un calcul de la valeur d'incrément ou de l'accroissement du signal durant un laps de temps fixe prédéterminé, typiquement la durée séparant la détermination de deux échantillons successifs), cette différentiation étant opérée avec limitation de pente, tant en valeur positive que négative, donnant ainsi un signal à pente bornée ou limitée, désigné SL. On explicitera plus bas la manière dont on peut réaliser cette limitation.

Dans un étage 16, le signal limité SL est soustrait du signal numérisé SN pour donner un signal traité ST qui est comparé, dans un étage 18, à un seuil de détection SD. On obtient ainsi un signal de commande SC.

Le seuil SD peut être un seuil fixe aussi bas que nécessaire, car seules les ondes caractéristiques sont présentes dans le signal traité ST.

Un autre avantage de l'invention est de permettre la modification du traitement opéré en modifiant quelques paramètres quantitatifs tels que les bornes limites (pente discriminante PD) de l'étage 14 ou le seuil de détection SD, soit sur commande, soit de façon entièrement automatique ; dans ce dernier cas, on réalise une auto-adaptation du circuit en fonction des caractéristiques du signal d'entrée numérique SN, notamment l'amplitude moyenne de celui-ci.

On va maintenant décrire une forme logicielle de mise en oeuvre de l'invention, en référence à l'organigramme de la figure 2, qui représente essentiellement (étages 20 à 30) les fonctions mises en oeuvre par l'étage 14 de la figure 1).

Sur cette figure, les blocs 20 à 30 servent à produire le signal limité courant SLᵢ (valeur à l'instant i) à partir de la valeur du signal numérisé SNᵢ, pour obtenir le signal traité STᵢ.

Après l'étape d'initialisation 20, à l'étape 22 on compare le signal numérisé courant SNᵢ à la valeur précédente SLᵢ₋₁ du signal limité et on examine si, en valeur absolue, leur différence est strictement supérieure à la pente discriminante PD.

Dans la négative, la pente de variation du signal est donc inférieure à la pente discriminante et le signal limité ne subit aucune modification et prend la valeur du signal numérisé (étape 26), qu'il suit donc exactement.

Dans le cas contraire, ceci signifie que le signal numérisé SNᵢ a varié très vite, et le signal limité SL ne va donc suivre le signal numérisé qu'après limitation des pentes aux valeurs PSC et PSD, respectivement en croissance et en décroissance.

Le test 24 suivant détermine si le signal croît ou décroît. En cas de décroissance (bloc 28), on donne au signal limité courant SLᵢ sa valeur précédente SLᵢ₋₁ diminuée de la pente de suivi en décroissance PSD. En cas de croissance (bloc 30), on donne au signal limité courant SLᵢ sa valeur précédente SLᵢ₋₁ augmentée de la pente de suivi en croissance PSC.

Enfin, on calcule (bloc 32) la valeur du signal traité courant STᵢ, qui est simplement la différence entre la valeur du signal numérisé SNᵢ et la valeur du signal limité SLᵢ (fonction mise en oeuvre à l'étage 16 de la figure 1).

On notera que la différence ainsi calculée est nulle en cas de test négatif à l'étape 22, c'est-à-dire si l'on est passé par l'étape 26. Si le signal numérisé a une pente inférieure à la pente discriminante, la valeur du signal traité sera nulle, et donc, si l'on choisit de façon judicieuse la valeur de PD, les signaux parasites de pente faible seront totalement éliminés du signal traité, et non simplement atténués comme le ferait un filtrage.

En ce qui concerne les pentes de suivi en croissance et en décroissance PSC et PSD, elles devront être choisies suffisamment faibles pour ne pas trop atténuer les signaux caractéristiques rapides, mais pas trop faibles car elles conditionnent en fait la rapidité du signal traité à rattraper le signal numérisé en cas de divergence, notamment à la mise en route du système.

On peut ainsi par exemple égaler les valeurs des pentes PSC et PSD et indexer ces valeurs sur la pente discriminante, en prenant par exemple PSC = PSD = PD/4. En ce qui concerne le choix de la valeur de la pente discriminante, celle-ci peut résulter d'une étude statistique, la valeur d'un coefficient de 50 mV/s par millivolt de signal cardiaque étant une valeur typique permettant une bonne réjection des signaux parasites.

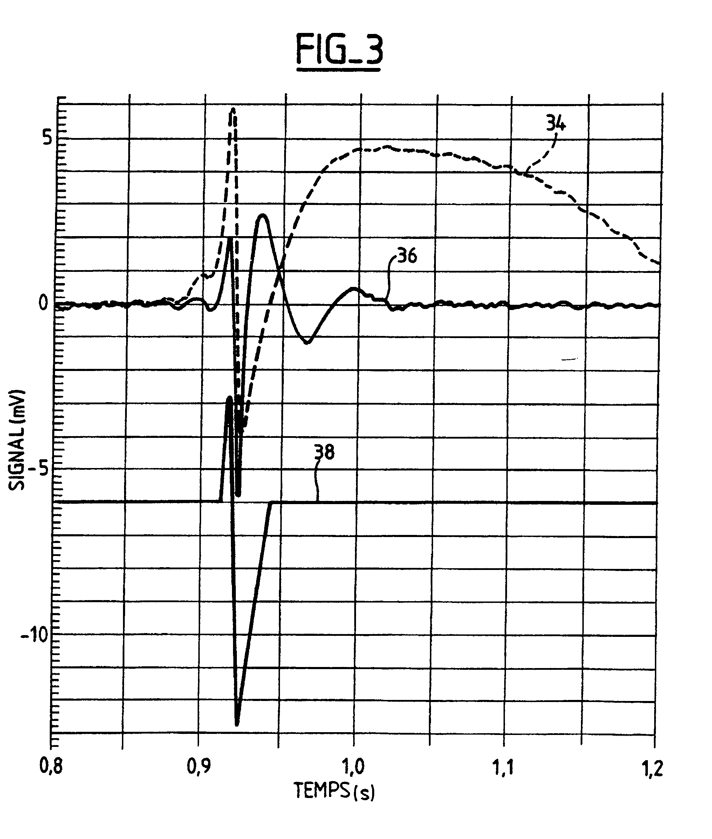

La figure 3 montre un exemple de résultat comparatif obtenus grâce à l'invention.

La référence 34 illustre un signal ventriculaire spontané, avec une onde R (caractéristique) et une onde T (parasite). En 36, on a illustré le signal procuré par un filtrage classique passe-haut à 16 Hz (filtre de Tchebyscheff d'ordre 3, ondulation 0,1 dB).

Ce résultat est à comparer au signal 38 obtenu par la mise en oeuvre de l'invention, où l'on voit clairement que ce signal ne comporte que le complexe R caractéristique dont, précisément, le dispositif cherche à détecter la survenance (dans cet exemple, la pente discriminante utilisée est de 0,45 V/s, le signal de l'onde R ayant une amplitude de 10 mV).

Le signal parasite constitué par l'onde T ainsi que le léger bruit observé sur le signal d'entrée ne laissent aucun résidu, à la différence du signal simplement filtré en 36 ; le bruit d'amplitude modérée étant éliminé du fait qu'il présente une pente inférieure à la pente discriminante.

Ceci permet de détecter l'onde R en plaçant le seuil de détection à un niveau très faible, par exemple de 0,4 mV, sans craindre de fausse détection. Enfin, on notera que l'amplitude de l'onde R est moins atténuée par le traitement de l'invention que par le filtrage.

## Revendications

1. Un dispositif médical implantable actif, notamment un stimulateur cardiaque de type sentinelle, comportant :
- des moyens détecteurs (10, 12), pour recueillir le signal cardiaque (SA), spontané ou stimulé, du porteur du dispositif, ce signal cardiaque recueilli comportant une composante de signal caractéristique et des composantes de signaux parasites indésirables, et
- des moyens discriminateurs (14-16), recevant en entrée le signal cardiaque et délivrant en sortie un signal (ST) correspondant essentiellement au signal caractéristique débarrassé des signaux parasites,
dispositif **caractérisé en ce que** les moyens discriminateurs comportent des moyens différentiateurs et limiteurs (14) pour évaluer des variations successives du signal cardiaque échantillonné de façon appropriée et délivrer en sortie soit un signal correspondant au signal cardiaque courant si la variation ainsi évaluée est comprise entre des limites positive et négative prédéterminées, soit un signal cardiaque dont la variation est bornée à une valeur prédéterminée, respectivement positive (PSC) ou négative (PSD), dans le cas contraire.

2. Le dispositif de la revendication 1, dans lequel :
- les moyens détecteurs comportent des moyens (12) pour échantillonner et numériser le signal cardiaque détecté, et
- les moyens différentiateurs et limiteurs (14) établissent les valeurs successives de pente du signal cardiaque par évaluation d'incréments entre deux valeurs d'échantillonnage successives.

3. Le dispositif de la revendication 2, dans lequel les moyens différentiateurs et limiteurs (14) retranchent du signal numérisé courant non borné (SNᵢ) la valeur précédente éventuellement bornée de ce signal (SLᵢ₋₁) et comparent la valeur absolue de la différence résultante à une valeur prédéterminée de pente discriminante (PD).

4. Le dispositif de la revendication 3, dans lequel, lorsque la valeur absolue de la différence résultante est supérieure à la valeur de pente discriminante (PD), les moyens différentiateurs et limiteurs ajoutent ou retranchent, selon le cas, à la valeur précédente, éventuellement bornée, du signal numérisé courant (SLᵢ₋₁) un incrément donné égal à ladite valeur prédéterminée positive ou négative (PSC, PSD) et délivrent en sortie une valeur traitée correspondante (STᵢ).

5. Le dispositif de la revendication 1, dans lequel la limite positive (PSC) et/ou la limite négative (PSD) des moyens limiteurs de pente sont des valeurs ajustables, notamment en fonction de l'évolution du signal cardiaque détecté.

6. Le dispositif de la revendication 3, dans lequel la valeur de pente discriminante (PD) est une valeur ajustable, notamment en fonction de l'évolution du signal cardiaque détecté.

## Patentansprüche

1. Aktive, implantierbare medizinische Vorrichtung, insbesondere Herzschrittmacher vom Typ Überwachung, aufweisend:
- Detektormittel (11, 12) zum Empfangen des spontanen oder stimulierten Herzsignals (SA) des Trägers der Vorrichtung, wobei dieses empfangene Herzsignal eine charakteristische Signalkomponente und unerwünschte, parasitäre Signalkomponenten aufweist, und
- Diskriminatormittel (14-16), welche am Eingang das Herzsignal empfangen und am Ausgang ein Signal (ST) liefern, welches im Wesentlichen dem charakteristischen Signal entspricht, von welchem parasitäre Signale entfernt wurden,
wobei die Vorrichtung **dadurch gekennzeichnet ist, dass** die Diskriminatormittel Differenzier- und Limitiermittel (14) aufweisen zum Bewerten von aufeinander folgenden Variierungen des als Stichprobe genommenen Herzsignals in einer angepassten Weise und Liefern am Ausgang entweder ein Signal entsprechend dem laufenden Herzsignal, wenn die so bewertete Variierung zwischen vorbestimmten positiven und negativen Grenzen enthalten ist, oder ein Herzsignal, dessen Variierung auf einen vorbestimmten Wert begrenzt ist, jeweils positiv (PSC) oder negativ (PSD) im umgekehrten Fall.

2. Vorrichtung nach Anspruch 1, in welcher:
- die Detektiermittel Mittel (12) zur Stichprobenentnahme und Digitalisieren des detektierten Herzsignals aufweisen, und
- Differenzier- und Limitiermittel (14) die aufeinander folgenden Abfallwerte des Herzsignals aufstellen durch Bewertung eines Inkrements zwischen zwei aufeinander folgenden Stichprobenwerten.

3. Vorrichtung nach Anspruch 2, in welcher die Differenzier- und Limitiermittel (14) vom laufenden, nicht begrenzten, digitalisierten Signal (SNᵢ) den vorherigen, gegebenenfalls begrenzten Wert dieses Signals (SLᵢ₋₁) abziehen und den absoluten Wert der resultierenden Differenz mit einem vorbestimmten, diskriminierenden Abfallwert (PD) vergleichen.

4. Vorrichtung nach Anspruch 3, in welcher, wenn der resultierende absolute Differenzwert größer als der diskriminierte Abfallwert (PD) ist, die Differenzier- und Limitiermittel je nach Fall zu dem vorherigen Wert des digitalisierten, laufenden Signals (SLᵢ₋₁), gegebenenfalls begrenzt, ein gegebenes Inkrement hinzufügen oder abziehen, welches gleich ist zu dem positiven oder negativen vorherbestimmten Wert (PSC, PSD) und am Ausgang einen entsprechenden behandelten Wert (STᵢ) liefern.

5. Vorrichtung nach Anspruch 1, in welcher die positive Grenze (PSC) und/oder die negative Grenze (PSD) von den Limitiermitteln eines Abfalls einstellbare Werte sind, insbesondere in Abhängigkeit von der Entwicklung des detektierten Herzsignals.

6. Vorrichtung nach Anspruch 3, in welcher der diskriminierende Abfallwert eines Abfalls (PD) ein einstellbarer Wert ist, insbesondere in Abhängigkeit von der Entwicklung des detektierten Herzsignals.

## Claims

1. An active implantable medical device, in particular a sentinel-type cardiac pacemaker, comprising:
- detecting means (10, 12) for collecting the spontaneous or stimulated cardiac signal (SA) of the bearer of the device, said collected cardiac signal comprising a characteristic signal component and components of undesirable parasitic signals, and
- discriminating means (14-16), receiving as an input the collected cardiac signal and delivering as an output a signal (ST) essentially corresponding to the characteristic signal from which parasitic signals have been removed,
said device being **characterized in that** the discriminating means include differentiating and limiting means (14) for evaluating successive variations of the collected, suitably sampled, cardiac signal and delivering as an output either a signal corresponding to the instant cardiac signal if the difference so evaluated is comprised between positive and negative predetermined limits, or a cardiac signal the variation of which is limited to a predetermined, respectively positive (PSC) or negative (PSD), value in the opposite case.

2. The device of claim 1, wherein :
- the detecting means include means (12) for sampling and digitizing the collected cardiac signal, and
- the differentiating and limiting means (14) establish the successive slope values of the cardiac signal by evaluating increments between two successive sampling values.

3. The device of claim 2, wherein the differentiating and limiting means (14) subtract from the digitized, non-limited instant signal (SNi) the preceding, possibly limited, value (SLᵢ₋₁) of this signal and compare the absolute value of the resulting difference to a predetermined, discriminating-slope value (PD).

4. The device of claim 2, wherein, when the absolute value of the resulting difference is greater than the discriminating-slope value (PD), the differentiating and limiting means (14) add or subtract, depending on the case, to the preceding, possibly limited, value of the instant digitized signal (SLᵢ₋₁) a given increment equal to said predetermined, positive or negative, value (PSC, PSD) and deliver as an output a corresponding processed value (STᵢ).

5. The device of claim 1, wherein, when the positive (PSC) and/or negative (PSD) limit of the slope-limiting means are adjustable values, in particular as a function of the evolution of the collected cardiac signal.

6. The device of claim 3, wherein, when the discriminating-slope value (PD) is an adjustable value, in particular as a function of the evolution of the collected cardiac signal.
